# EUROPEAN PATENT SPECIFICATION

(11) **EP 0 949 240 B1**
(45) Date of publication and mention of the grant of the patent: **22.01.2003**
(21) Application number: 98302258.3
(22) Date of filing: 25.03.1998
(51) Int. Cl.: C07C 201/08, C07C 205/11, C07C 205/12

(54) **A process for the production of nitroaromatic compounds from aromatic hydrocarbons using modified clay catalysts**
Verfahren zur Herstellung von nitroaromatischen Verbindungen aus aromatischen Kohlenwasserstoffen durch Verwendung von modifizierten Tonkatalysatoren
Procédé pour la préparation de composés nitro aromatiques à partir d'hydrocarbures aromatiques utilisant des catalyseurs d'argile modifiés

(43) Date of publication of application: 13.10.1999
(73) Proprietor: Council of Scientific and Industrial Research, New Delhi 110 001 (IN)
(72) Inventor: Choudary, Boyapati Manoranjan, Andhra Pradesh (IN); Kantam, Mannepalli Lakshmi, Andhra Pradesh (IN); Sateesh, Mutyala, Andhra Pradesh (IN); Rao, Kottapalli Koteswara, Andhra Pradesh (IN); Raghavan, Kondapuram Vijaya, Andhra Pradesh (IN)
(74) Representative: Lamb, Martin John Carstairs

(56) References cited:
- EP-A- 0 343 048
- GB-A- 2 000 141
- B.M. CHOUDARY ET AL.: "Fe3+ Montmorillonite catalyst for selective nitration of chlorobenzene" JOURNAL OF MOLECULAR CATALYSIS, vol. 87, no. 1, 19 January 1994, AMSTERDAM, pages 33-38, XP002074615
- M.H. GUBELMANN ET AL.: "Nitric acid associated with inorganic solids" HETEROGENEOUS CATALYSIS AND FINE CHEMICALS II, vol. 59, 1990, AMSTERDAM-OXFORD-NEW YORK-TOKYO, pages 471-478, XP002074616
- DATABASE WPI Section Ch, Week 7629 Derwent Publications Ltd., London, GB; Class E14, AN 76-54940X XP002074618 & JP 51 063 134 A (TEIJIN LTD)
- ANDR CORNELIS ET AL. : "Regioselective liquid-phase toluene nitration with modified clays as catalysts" CHEMISTRY LETTERS., 1988, TOKYO JP, pages 1839-1842, XP002074617

## Description

This invention relates to a process for the production of nitro aromatic compounds from aromatic hydrocarbons using modified clay catalysts. This invention particularly relates to a process for nitration of aromatic hydrocarbons using modified clay catalysts dispensing the use of H₂SO₄. Thus, this process totally eliminates disposal of salts formed consequent to the neutralisation of sulphuric acid.

Nitration has been an active area of industrial chemistry for over a century Nitration process is used for the production of many large-volume chemicals such as nitrobenzene, nitrotoluenes and nitrochlorobenzenes, and other nitroaromatics. These nitroaromatics are vital intermediates for dyes, pharmaceuticals, perfumes and pesticides.

Nitration of aromatic hydrocarbons is performed classically with a mixture of nitric and sulfuric acids. One of the major disadvantages of this method is formation of byproducts of polynitration and also causing environmental pollution during disposal of spent acid.

In the past, several methods were proposed for the nitration of aromatic hydrocarbons. For example, a US patent (U S 3,981,935; 21 September 1976) discloses a two stage continuous process for benzene mononitration with HNO₃ and H₂SO₄. Another Japanese patent (Jpn Kokai Tokkyo Koho JP 8224, 331; 08 Feb 1982) gives a method in which chlorobenzene is nitrated with a mixture of HNO₃ and H₂SO₄ at 85-150°C (p: 61.45 : 37.8). A European patent (EP 675,104, 4 October, 1995) describes a method for nitration of chlorobenzene at 60-160 °C using HNO₃ and H₂SO₄. Another Japanese patent (Jpn Kokai Tokkyo Koho JP 05,170,706, 09 July, 1993) describes a method for nitration of toluene with HNO₃ and H₂SO₄ at 0 - 50 °C (o:m:p 55 : 2.3 : 38.9). However, all these methods have a common disadvantage such as use of hazardous H₂SO₄, whose disposal poses a significant environmental problem. Although it has been known for some time that benzene and its homologs can be nitrated with HNO₃ alone, little or no progress has been made in this direction. The disadvantage in this method is the use of large excess of nitric acid (molar ratio of nitric acid to benzene are 2:1 to 4:1) increase the possibility of poly-nitro compounds formation and affect the economics of the project.

Several catalytic methods are also known for nitration of aromatic hydrocarbons. Although these methods are practised in laboratories, any of these methods has no feasibility on a commercial basis in terms of economics and other factors. For example, a US patent (U.S., 4,234,470, 18 November, 1980) described a method for nitration of benzene, chlorobenzene and toluene with HNO₃ in presence of Nafion catalyst This method employs expensive Nafion resin whose activity is decreasing on each cycle.

Recently attention has been focused on the development of environmentally friendly solid acid catalysts such as zeolites, sulfated zirconia and Nafion especially in Friedel-Crafts reactions to replace environmentally hazardous chemicals, anhydrous aluminium chloride and sulfuric acid respectively in alkylation and nitration reactions.

Vapour phase methods of nitrating aromatic compounds are described in EP-A-0343048 and GB 2000141.

Methods of nitrating aromatic compounds using acetic anhydride are described in Journals of Molecular Catalysis 87 (1994) 33-38 (Choudray et al), and Chemistry Letters 1839 - 1842, 1988 (Cornelis et al).

Acid clay catalysts are used to nitrate aromatic compounds in Heterogeneous Catalysis and Fine Chemical II, 1991, p471 - 478 (Gubelmann et al), and JP 51063134. The main objective of the present invention is the use of cheaply available modified montmorillonite, a smectite clay, as a solid acid catalyst in the nitration of aromatic hydrocarbons, without using acetic anhydride. Cation exchange of interstitial cations with transitions metal ions boosts the Lewis acidity.

The main objective of the present invention is the use of cheaply available modified montmorillonite, a smectite clay as a solid acid catalyst in the nitration of aromatic hydrocarbons, without using acetic anhydride. Cation exchange of interstitial cations with transition metal ions boosts the Lewis acidity.

The present invention provides a process for preparation of nitro aromatic compounds from aromatic hydrocarbons using modified clay catalysts which comprises: nitrating aromatic hydrocarbons in liquid phase using fuming nitric acid in the molar ratio of nitric acid to aromatic hydrocarbon of 0.3:1 to 1.2:1 in the presence of metal exchanged clay at a temperature range between 25 ° - 155°C for 0.25 to 2.0 hours in the absence of acetic anhydride, and recovering corresponding nitro aromatic compounds. Conventional methods may be used to recover the nitro compounds, such as herein described and as in the examples.
The clay catalyst used is preferably a metal ion exchanged clay.
Metal ions used are selected from Al³⁺, La^{3+,} Cu²⁺, Fe³⁺ and Zn²⁺. The amount of nitric acid used preferably ranges from 4 ml (84 mmol) to 17 ml (357 mmol).
Aromatic hydrocarbons used are preferably selected from benzene, chlorobenzene, toluene, o-xylene, m-xylene, p-xylene, anisole and naphthalene.
Recovery of nitroarenes is suitably carried out by separating the catalyst by filtration and removing the excess aromatic hydrocarbons by distillation or by evaporation for example in a rotary evaporator or rotavapor.

Preferably, the process is effected by continuous removal of water formed by the reaction using a Dean-Stark apparatus.

The process of the present invention is illustrated with reference to the following non-limiting examples.

Metal-exchanged montmorillonite catalysts were prepared as described in example 1 and employed in the nitration reactions on aromatic compounds, examples 2-21.

### Example 1

A series of catalysts were prepared
**a) *K10 montmorillonite -*** Montmorillonite employed in the synthesis was obtained from Fluka (Grade K10) with exchange capacity of 0.8 equi..
**b) *Kunipia clay -*** Japanese clay (e.c., 1.15 equi.) was taken as it is without any modification.
**c)** i) ***Fe***^{***3***}^{**+**}***-exchanged montmorillonite catalyst :*** To a 1 It stirred aqueous solution of FeCl₃ (1.0 M), 80 g of K10-montmorillonite was added. Stirring was maintained for 16-30 hrs in order to saturate the exchange capacity of montmorillonite K10. The clay suspension was centrifuged and the supernatant solution was discharged The clay catalyst was filtered, and washed with distilled water and the washing cycles were repeated until disappearance of Cl⁻ions from the discarded water. The clay was dried overnight in an oven at 120°C and finely ground in a mortar
   ii) Fe³⁺ exchanged Kunipia clay. It was prepared as in example (c) taking Kunipia clay instead of K10 montmorillonite.
d) ***Al***^{***3+***} ***- exchanged catalyst* Al**^{**3+**} **-** exchanged catalyst was prepared in the same manner as in example c stirring 1 It aqueous solution of AlCl₃ ( 1.0 M) and 80 g of K10-montmorillonite.
e) ***Zn***^{***2***}^{**+**} ***-exchanged catalyst*** It was prepared in the same manner as in example c stirring 1M solution of ZnCl₂ and 80 g of K10 montmorillonite.
**f) *Cu***^{***2***}^{**+**} ***-exchanged catalyst*** It was prepared in the same manner as in example c stirring 1M solution of CuCl₂ and 80 g of K10 montmorillonite
**g) *La***^{**3+**} ***-exchanged catalyst*** It was prepared in the same manner as in example c stirring 1M solution of LaCl₃ and 80 g of K10 montmorillonite
**h) *FePILC** *from K10 montmorillonite*** The Na-montmorillonite was prepared by suspending and stirring commercial K10 in excess aqueous sodium chloride solution for 24 h. The Na-montmorillonite was separated by centrifugation and washed free of chloride ions by deionised distilled water and air dried The cation exchange capacity of the air-dried clay is 0.8 equi Trinuclear acetato hydroxy-iron (III) nitrate, Fe₃(OCOCH₃)₇OH 2H₂ONO₃ was used as the cation source for exchanging with the Na-montmorillonite It was prepared by dissolving 80 8g of Fe(NO₃)₃ 5H₂O in 50 ml of ethyl alcohol. This was reacted with 140 ml acetic anhydride with the evolution of heat. The solution was then cooled in an ice bath and the resulting precipitate was separated and used without further purification in the pillaring procedure.

* Iron pillared clay

### Cation-exchange

A 0.04mole/l aqueous solution of trinuclear acetato-hydroxy-iron (III) nitrate (19.48 g) in 700 ml water was added to a stirred 1% Na-montmorillonite aqueous suspension (8 g of Na-mont in 800 ml of water) The mixture was stirred for 3hrs at 40°C, separated by centrifugation and washed with water several times to remove excess iron ions and dried in air
i) ***FePILC from Kunipia clay*** It was prepared as in example (h) taking Kunipia clay instead of K10 montmorillonite

### Example 2 (According to the Invention)

A mixture of benzene (25 ml) and Fe³⁺ \ Zn²⁺ montmorillonite (0.5g) were taken in a 50 ml two-necked round-bottomed flask equipped with Dean-Stark apparatus. Fuming nitric acid (5 ml) was added dropwise into the reaction mixture. The reaction mixture was heated to reflux where upon the required amount of liberated water was collected in the Dean-Stark apparatus which usually takes 1.5 hr. Later on, the catalyst was filtered and the reaction mixture was concentrated to obtain the pure mononitrobenzene.

### Example 3 (According to the Invention)

A mixture of benzene (25 ml) and Zn²⁺ montmorillonite catalyst (0.5g), were stirred in a 50 ml two-necked round bottomed flask. Fuming nitric acid (5ml) was added dropwise into the reaction mixture at room temperature and stirred for 1.5 hr. Later on, the catalyst was filtered and the reaction mixture was concentrated to obtain the pure mononitrobenzene.

### Example 4 (According to the Invention)

A mixture of benzene (6 ml) and Zn²⁺ montmorillonite catalyst (2 g, excess catalyst), were stirred in a 50 ml two-necked round bottomed flask. Fuming nitric acid (4 ml) was added dropwise into the reaction mixture at r.t. and stirred for 1.5 hrs. Later on, the catalyst was filtered and the reaction mixture was concentrated to obtain the pure mononitrobenzene.

### Example 5 (According to the Invention)

A mixture of benzene (25 ml) and Zn²⁺ montmorillonite (0.5g) were taken in a 50 ml two-necked round bottomed flask equipped with a Dean-Stark apparatus. Fuming nitric acid (17 ml) was added dropwise into the reaction mixture and the reaction mixture was heated to reflux where upon the required amount of liberated water is collected in the Dean Stark apparatus which usually take 2hrs. Later on, the catalyst was filtered and the reaction mixture is concentrated to obtain nitrobenzene.

### Example 6 (Comparative)

Benzene (25 ml) was taken in a 50 ml two-necked round bottomed flask equipped with a Dean-Stark apparatus. Fuming nitric acid (17 ml) was added dropwise into the reaction mixture and the reaction mixture was heated to reflux where upon the required amount of liberated water is collected in the Dean Stark apparatus which usually take 2hrs. Later on, the catalyst was filtered and the reaction mixture was concentrated to obtain nitrobenzene.

### Example 7 (Comparative)

Benzene (25 ml) was stirred in a 50 ml two-necked round bottomed flask. Fuming nitric acid (5ml) was added dropwise into the reaction mixture at r.t. and stirred for 1.5 hr. Then, the reaction mixture was concentrated to obtain the pure mononitrobenzene

### Example 8 (Comparative)

Benzene (25 ml) was taken in a 50 ml two-necked round-bottomed flask equipped with Dean-Stark apparatus. Fuming nitric acid (5 ml) was added dropwise into the reaction mixture. The reaction mixture was heated to reflux where upon the required amount of liberated water is collected in the Dean-Stark apparatus which usually takes 1.5hr. Then the reaction mixture was concentrated to obtain the pure mononitrobenzene.

### Example 9 (According to the Invention)

A mixture of toluene (25 ml) and Fe³⁺ \ Zn²⁺ montmoriilonite (0.5g) were taken in a 50 ml two-necked round-bottomed flask equipped with Dean-Stark apparatus. Fuming nitric acid (5 ml) was added dropwise into the reaction mixture. The reaction mixture was heated to reflux where upon the required amount of liberated water was collected in the Dean-Stark apparatus which usually takes 1.5hr. Later on, the catalyst was filtered and the reaction mixture was concentrated to obtain the mixture of nitrotoluenes

### Example 10 (According to the Invention)

A mixture of o-xylene (25 ml) and Fe³⁺ \ Zn²⁺ montmoriilonite (0.5g) were taken in a 50 ml two-necked round-bottomed flask equipped with Dean-Stark apparatus. Fuming nitric acid (5 ml) was added dropwise into the reaction mixture. The reaction mixture was heated to reflux where upon the required amount of liberated water was collected in the Dean-Stark apparatus which usually takes 1.5hr. Later on, the catalyst was filtered and the reaction mixture was concentrated to obtain the mixture of nitro o-xylenes.

### Example 11 (According to the Invention)

A mixture of m-xylene (25 ml) and Fe³⁺ \ Zn²⁺ montmoriilonite (0.5g) were taken in a 50 ml two-necked round-bottomed flask equipped with Dean-Stark apparatus. Fuming nitric acid (5 ml) was added dropwise into the reaction mixture. The reaction mixture was heated to reflux where upon the required amount of liberated water was collected in the Dean-Stark apparatus which usually takes 1.5hr. Later on, the catalyst was filtered and the reaction mixture was concentrated to obtain the mixture of nitro m-xylenes.

### Example 12 (According to the Invention)

A mixture of p-xylene (25 ml) and Fe³⁺ \Zn²⁺ montmoriilonite (0.5g) were taken in a 50 ml two-necked round-bottomed flask equipped with Dean-Stark apparatus. Fuming nitric acid (5 ml) was added dropwise into the reaction mixture. The reaction mixture was heated to reflux where upon the required amount of liberated water was collected in the Dean-Stark apparatus which usually takes 1.5hr. Later on, the catalyst was filtered and the reaction mixture was concentrated to obtain the nitro p-xylene.

### Example 13 (According to the Invention)

A mixture of anisole (25 ml) and Fe³⁺\ Zn²⁺ montmoriilonite (0.5g) were taken in a 50 ml two-necked round-bottomed flask equipped with Dean-Stark apparatus. Fuming nitric acid (5 ml) was added dropwise into the reaction mixture. The reaction mixture was heated to reflux where upon the required amount of liberated water was collected in the Dean-Stark apparatus which usually takes 1.5hr. Later on, the catalyst was filtered and the reaction mixture was concentrated to obtain the mixture of nitro anisoles

### Example 14 (According to the Invention)

A mixture of chlorobenzene (25 ml) and Fe³⁺\ Zn²⁺ montmoriilonite (0.5g) were taken in a 50 ml two-necked round-bottomed flask equipped with Dean-Stark apparatus. Fuming nitric acid (5 ml) was added dropwise into the reaction mixture. The reaction mixture was heated to reflux where upon the required amount of liberated water was collected in the Dean-Stark apparatus which usually takes 1.5hr. Later on, the catalyst was filtered and the reaction mixture was concentrated to obtain the mixture of nitrochlorobenzenes.

### Example 15 (According to the Invention)

A mixture of chlorobenzene (25 ml) and Zn²+ montmorillonite catalyst (0.5g), were stirred in a 50 ml two-necked round bottomed flask. Fuming nitric acid (5ml) was added dropwise into the reaction mixture at r.t. and stirred for 1.5 hr. Later on, the catalyst was filtered and the reaction mixture was concentrated to obtain the mixture of nitrochlorobenzenes

### Example 16 (According to the Invention)

A mixture of chlorobenzene (6 ml) and Zn²⁺ montmorillonite catalyst (2 g, excess catalyst), were stirred in a 50 ml two-necked round bottomed flask. Fuming nitric acid (4 ml) was added dropwise into the reaction mixture at r.t. and stirred for 1.5 hrs. Later on, the catalyst was filtered and the reaction mixture was concentrated to obtain the mixture of nitrochlorobenzenes.

### Example 17 (According to the Invention)

A mixture of chlorobenzene (25 ml) and Zn²⁺ montmorillonite (0.5g) were taken in a 50 ml two-necked round bottomed flask equipped with a Dean-Stark apparatus. Fuming nitric acid (17 ml) was added dropwise into the reaction mixture and the reaction mixture was heated to reflux where upon the required amount of liberated water is collected in the Dean Stark apparatus which usually take 2hrs. Later on, the catalyst was filtered and the reaction mixture was concentrated to obtain the mixture of nitrochlorobenzenes.

### Example 18 (Comparative)

Chlorobenzene (25 ml) was taken in a 50 ml two-necked round bottomed flask equipped with a Dean-Stark apparatus. Fuming nitric acid (17 ml) was added dropwise into the reaction mixture and the reaction mixture was heated to reflux where upon the required amount of liberated water is collected in the Dean Stark apparatus which usually take 2hrs. Later on, the catalyst was filtered and the reaction mixture was concentrated to obtain the mixture of nitrochlorobenzenes.

### Example 19 (Comparative)

Chlorobenzene (25 ml) was stirred in a 50 ml two-necked round bottomed flask. Fuming nitric acid (5ml) was added dropwise into the reaction mixture at r.t. and stirred for 1.5 hr. Then, the reaction mixture was concentrated to obtain the mixture of nitrochlorobenzenes.

### Example 20 (Comparative)

Chlorobenzene (25 ml) was taken in a 50 ml two-necked round-bottomed flask equipped with Dean-Stark apparatus. Fuming nitric acid (5 ml) was added dropwise into the reaction mixture. The reaction mixture was heated to reflux where upon the required amount of liberated water is collected in the Dean-Stark apparatus which usually takes 1.5hr. Then the reaction mixture was concentrated to obtain the mixture of nitrochlorobenzenes.

### Example 21 (According to the Invention)

Naphthalene(13.62 g), Zn²⁺ Mont (0.25 g) and CCl₄ were stirred in 50 ml two necked round-bottomed flask equipped with Dean-Stark apparatus. Fuming nitric acid (5 ml) was added dropwise into the reaction mixture. The reaction mixture was heated to reflux where upon the required amount of liberated water is collected in the Dean-Stark apparatus which usually takes 1hr. Later on, the catalyst was filtered and the organic layer is concentrated to obtain 1-nitronaphthalene.
Yield: - 74%
The results are given in Table 1, Table 2 and Table 3

**TABLE 1**

| **Nitration of Chlorobenzene**^{**a**} **with various catalysts:-** | | | | | |
|---|---|---|---|---|---|
| **SI. No.** | **Catalyst** | **Temp (°C)** | **Conversion** **(%)** | **Isolated** **Yield (g)** | **Isomers**^{**b**} **(o/p)** |
| 1. | Al³⁺ Mont. | 145 | 48.3 | 8.12 | 37.4 : 62.6 |
| 2. | La³⁺ Mont. | 145 | 58.3 | 9.80 | 37.0 : 63.0 |
| 3. | Cu²⁺ Mont. | 145 | 58.8 | 9.88 | 37.4 : 62.6 |
| 4. | H⁺ Mont.* | 145 | 51.6 | 8.68 | 37.1 : 62.9 |
| 5. | K10 Mont.* | 145 | 64.2 | 10.80 | 37.3 : 62.7 |
| 6. | Fe³⁺ Mont. | 145 | 77.3 | 13.00 | 36.0 : 64.0 |
| 7. | Fe³⁺ Kunipia Mont. | 145 | 78.1 | 13.14 | 36.0 : 64.0 |
| 8. | Fe K10 PILC | 145 | 73.6 | 12.38 | 35.0 : 65.0 |
| 9. | Fe Kunipia PILC | 145 | 75.4 | 12.68 | 34.5 : 65.5 |
| 10. | Zn²⁺ Mont. | 145 | 82.2 | 13.83 | 38.0 : 62.0 |

| | | | | | |
|---|---|---|---|---|---|
| ^{a} The reactions were carried out as in example 14 by various metal catalysts | | | | | |
| ^{b} by NMR ; ratios of major isomers are given. | | | | | |
| *Comparative | | | | | |

**TABLE 2**

| **Nitration of Benzene and Chlorobenzene with various amounts of Fum. HNO**_{**3**}**:-** | | | | | | | |
|---|---|---|---|---|---|---|---|
| Sl. No. | Example^{a} | Aromatic | Amount of Acid (ml) | Catalyst | Temp (°C) | Conversion (%) | Isolated Yield (g) |
| 1. | 2 | Benzene | 5 | Zn²⁺ Mont. | 115 | 92.8 | 12.00 |
| 2. * | 8 | Benzene | 5 | Blank | 115 | 56.2 | 7.26 |
| 3. | 3 | Benzene | 5 | Zn²⁺ Mont. | R.T. | 47.8 | 6.17 |
| 4. * | 7 | Benzene | 5 | Blank | R.T. | 28.6 | 3.70 |
| 5. | 5 | Benzene | 17 | Zn²⁺ Mont. | 115 | 99.1^{c} | 39.50 |
| 6. * | 6 | Benzene | 17 | Blank | 115 | 65.8^{c} | 26.21 |
| 7. | 4 | Benzene | 4 | Zn²⁺Mont excess | R.T. | 82.0^{c} | 6.64 |
| 8. | 14 | Chlorobenzene | 5 | Zn²⁺ Mont. | 145 | 82.2 | 13.83 |
| 9. * | 20 | Chlorobenzene | 5 | Blank | 145 | 57.7 | 9.70 |
| 10. | 15 | Chlorobenzene | 5 | Zn²⁺ Mont. | R.T. | 61.4 | 10.33 |
| 11.* | 19 | Chlorobenzene | 5 | Blank | R.T. | 36.9 | 6.20 |
| 12. | 16 | Chlorobenzene | 17 | Zn²⁺ Mont. | 145 | 99.9^{c} | 38.70 |
| 13 * | 17 | Chlorobenzene | 17 | Blank | 145 | 61.4^{c} | 23.80 |
| 14. | 18 | Chlorobenzene | 4 | Zn²⁺ Mont excess | R.T. | 85.0^{c} | 7.09 |

| | | | | | | | |
|---|---|---|---|---|---|---|---|
| ^{a} as exemplified in the text ^{b} 5 ml of benzene was taken in Dean-Stark collector | | | | | | | |
| ^{c} based on aromatics by G.C. analysis | | | | | | | |
| *Comparative | | | | | | | |

**TABLE 3**

| **Nitration of Aromatics with Fum. HNO**_{**3**}**:-** | | | | | | | |
|---|---|---|---|---|---|---|---|
| Sl. No. | Example^{a} | Aromatic | Catalyst | Temp (°C) | Conversion (%) | Isolated yield (g) | Isomers (o/p) |
| 1 | 2 | Benzene | Fe³⁺-mont | 115 | 88.3 | 11.4 | -- |
| 2 | 2 | Benzene | Zn²⁺-mont | 115 | 92.8 | 12.0 | -- |
| 3 | 9 | Toluene | Fe³⁺-mont | 125 | 73.9 | 10.8 | 53.0 : 47.0 |
| 4 | 9 | Toluene | Zn²⁺-mont | 125 | 77.2 | 11.5 | 51.7 : 48.3 |
| 5 | 14 | Chlorobenzene | Fe³⁺-mont | 145 | 77.3 | 13.0 | 36.0 : 64.0 |
| 6 | 14 | Chlorobenzene | Zn²⁺-mont | 145 | 82.2 | 13.8 | 38.0 : 62.0 |
| 7 | 10 | o-Xylene | Fe³⁺-mont | 150 | 56.8 | 9.2 | 47.0^{c}: 53.0^{d} |
| 8 | 10 | o-Xylene | Zn²⁺-mont | 150 | 58.7 | 9.5 | 48.0^{c}: 52.0^{d} |
| 9 | 12 | p-Xylene | Fe³⁺-mont | 145 | 63.8 | 10.3 | -- |
| 10 | 12 | p-Xylene | Zn²⁺-mont | 145 | 68.1 | 11.0 | -- |
| 11 | 11 | m-Xylene | Fe³⁺-mont | 145 | 56.50 | 9.1 | 16.0^{e}: 84.0^{f} |
| 12 | 11 | m-Xylene | Zn²⁺-mont | 145 | 58.85 | 9.5 | 15.0^{e}: 85.0^{f} |
| 13 | 13 | Anisole | Fe³⁺-mont | 155 | 43.42 | 7.0 | 13.0 : 87.0 |
| 14 | 13 | Anisole | Zn²⁺-mont | 155 | 46.00 | 7.5 | 12.0: 88.0 |

| | | | | | | | |
|---|---|---|---|---|---|---|---|
| a: as exemplified in text. b: by NMR ; ratios of major isomers are given. | | | | | | | |
| c: 3-nitro-o-xylene, | | | | | | | |
| d: 4-nitro-o-xylene, | | | | | | | |
| e: 2-nitro-m-xylene, | | | | | | | |
| f: 4-nitro-m-xylene | | | | | | | |

The present process has several advantages as described below
1. An ecofriendly process for production of aromatic nitro compounds with comparable activity when compared with the other conventional processes
2. The support of the catalyst, clay is cheap and abundantly available in nature.
3. The use of sulphuric acid, a hazardous chemical is dispensed with
4. The use of an expensive acetic anhydride which acts as water scavenger and nitrating agent in some of the processes considered to he alternative is also dispensed with
5. The present process envisages no disposable problem as the catalyst can be used for several recycles. The catalyst was subjected to 4 cycles which displayed almost consistent activity.
6. The present process is environmentally safe since there is no effluent disposable problem.
7. Amount of HNO₃ used is 4 ml (84 mmol) to 17 ml (357 mmol)

## Claims

1. A process for preparation of nitro aromatic compounds from aromatic hydrocarbons using modified clay catalysts which comprises : nitrating aromatic hydrocarbons in liquid phase using fuming nitric acid in the molar ratio of nitric acid to aromatic hydrocarbon of 0.3:1 to 1.2:1 in the presence of metal exchanged clay at a temperature range between 25 ° - 155°C for 0.25 to 2.0 hrs in the absence of acetic anhydride and recovering corresponding nitro aromatic compounds.

2. A process as claimed in claim 1, wherein the clay catalyst used is metal ion exchanged clay.

3. A process as claimed in claim 2 wherein metal ions used are selected from Al³⁺, La³⁺, Cu²⁺, Fe³⁺ and Zn²⁺.

4. A process as claimed in Claim 3, wherein metal ions used are selected from Fe³⁺ and Zn²⁺.

5. A process as claimed in any of claims 1 to 4, wherein aromatic hydrocarbons used are selected from benzene, chlorobenzene, toluene, o-xylene, m-xylene, p-xylene, anisole and naphthalene.

6. A process as claimed in claim 1 to 5 wherein recovery of nitro aromatic compounds is carried out by separating the catalyst by filtration and removing the excess aromatic hydrocarbons by distillation or by concentration by evaporation.

## Patentansprüche

1. Verfahren zur Herstellung aromatischer Nitroverbindungen aus aromatischen Kohlenwasserstoffen unter Verwendung modifizierter Tonmineralkatalysatoren, mit den folgenden Schritten: Nitrieren aromatischer Kohlenwasserstoffe in der flüssigen Phase unter Verwendung von rauchender Salpetersäure im Molverhältnis Salpetersäure zu aromatischem Kohlenwasserstoff von 0,3:1 bis 1,2:1 über 0,25 bis 2,0 Stunden in Gegenwart eines metallsubstituierten Tonminerals in einem Temperaturbereich von 25° - 155°C, in Abwesenheit von Essigsäureanhydrid, und Rückgewinnung entsprechender aromatischer Nitroverbindungen.

2. Verfahren nach Anspruch 1, wobei der verwendete Tonmineralkatalysator ein Tonmineral mit ausgetauschten Metallionen ist.

3. Verfahren nach Anspruch 2, wobei die verwendeten Metallionen unter Al³⁺, La³⁺, Cu²⁺, Fe³⁺ und Zn²⁺ ausgewählt sind.

4. Verfahren nach Anspruch 3, wobei die verwendeten Metallionen unter Fe³⁺ und Zn²⁺ ausgewählt sind.

5. Verfahren nach einem der Ansprüche 1 bis 4, wobei die verwendeten aromatischen Kohlenwasserstoffe unter Benzol, Chlorbenzol, Toluol, o-Xylol, m-Xylol. p-Xylol, Anisol und Naphthalin ausgewählt sind.

6. Verfahren nach einem der Ansprüche 1 bis 5, wobei die Rückgewinnung der aromatischen Nitroverbindung durch Abtrennen des Katalysators mittels Filtration und Entfernen der überschüssigen Kohlenwasserstoffe durch Destillation oder Eindampfen ausgeführt wird.

## Revendications

1. Procédé pour la préparation de composés nitroaromatiques à partir d'hydrocarbures aromatiques en utilisant des catalyseurs d'argile modifiée qui comprend: la nitration d'hydrocarbures aromatiques en phase liquide en employant de l'acide nitrique fumant dans le rapport molaire de l'acide nitrique sur l'hydrocarbure aromatique de 0,3:1 à 1,2:1 en présence d'une argile ayant subi un échange de métal à un intervalle de température entre 25°C et 155°C pendant 0,25 à 2,0 h en l'absence d'anhydride acétique et la récupération des composés nitroaromatiques correspondants.

2. Procédé suivant la revendication 1, dans lequel le catalyseur d'argile utilisé est une argile ayant subi un échange d'ions métalliques.

3. Procédé suivant la revendication 2, dans lequel les ions métalliques utilisés sont choisis parmi Al³⁺, La³⁺, Cu²⁺, Fe³⁺ et Zn²⁺.

4. Procédé suivant la revendication 3, dans lequel les ions métalliques utilisés sont choisis parmi Fe³⁺ et Zn²⁺.

5. Procédé suivant l'une quelconque des revendications 1 à 4, dans lequel les hydrocarbures aromatiques utilisés sont choisis parmi le benzène, le chlorobenzène, le toluène, l'o-xylène, le m-xylène, le p-xylène, l'anisole et le naphtalène.

6. Procédé suivant les revendications 1 à 5, dans lequel la récupération des composés nitroaromatiques est réalisée en séparant le catalyseur par filtration et en retirant les hydrocarbures aromatiques en excès par distillation ou par concentration par évaporation.
